# EUROPEAN PATENT SPECIFICATION

(11) **EP 1 753 376 B1**
(45) Date of publication and mention of the grant of the patent: **02.01.2013**
(21) Application number: 05745282.3
(22) Date of filing: 04.05.2005
(51) Int. Cl.: A61F 2/44

(54) **ARTIFICIAL INTERVERTEBRAL DISC FOR LATERAL INSERTION**
KÜNSTLICHE BANDSCHEIBE ZUR SEITLICHEN EINFÜHRUNG
DISQUE INTERVERTEBRAL ARTIFICIEL POUR INSERTION LATERALE

(30) Priority: 05.05.2004 US 839100
(43) Date of publication of application: 21.02.2007
(73) Proprietor: Warsaw Orthopedic, Inc., Warsaw, IN 46581 (US)
(72) Inventor: PETERMAN, Marc, M., Austin, TX 78704 (US)
(74) Representative: Viering, Jentschura & Partner
(86) International application number: PCT/US2005/015581
(87) International publication number: WO 2005/107656

(56) References cited:
- EP-A- 1 103 237
- WO-A-02/47587
- WO-A-2004/071359
- FR-A- 2 734 148
- US-A1- 2003 208 273

## Description

### Background

The present disclosure relates generally to the field of orthopedics and spinal surgery, and in some embodiments, the present disclosure relates to artificial intervertebral discs for lateral insertion.

In the treatment of diseases, injuries or malformations affecting spinal motion segments, and especially those affecting disc tissue, it has long been known to remove some or all of a degenerated, ruptured or otherwise failing disc. In cases involving intervertebral disc tissue that has been removed or is otherwise absent from a spinal motion segment, corrective measures are taken to ensure the proper spacing of the vertebrae formerly separated by the removed disc tissue. In some instances, prosthetic devices are inserted into the disc space to maintain the structural integrity of the spinal column.

Insertion of prosthetic devices has heretofore been accomplished from an anterior approach to the vertebrae. However, in some regions of the spine, correction from the anterior approach may present difficulties due to the presence of important anatomical structures such as vessels and nerves. For example, the straight anterior approach to the disc space between vertebra L4 and L5, as well as the superior disc levels, can present high surgical risks during the insertion of a prosthetic device because of the attachment of the major vessels to the anterior aspect of the spine. Alternatives to correction from an anterior approach are therefore desirable.

Moreover, subsidence of prosthetic devices into adjacent vertebrae has often been a problem due to insufficient contact between the prosthetic device and the appropriate bearing surface provided by the adjacent vertebrae. For example, subsidence of the prosthetic device into the adjacent vertebrae may occur, which can result in a decreased amount of support offered by the prosthetic device.

Therefore, what is needed is an artificial intervertebral prosthetic device, which can be inserted from the lateral approach. Furthermore, an artificial intervertebral prosthetic device is needed whereby the lateral window associated with the lateral insertion of the disc is minimized and the bearing contact between the device and the adjacent vertebrae is increased.

WO 2004/071359 A1 which falls under Art 54(3) EPC discloses a prosthetic device for lateral insertion into an intervertebral space, comprising a first component having a first flange for engaging a first vertebra from a lateral approach, the first component having a first articular surface, and a second component having a second flange for engaging a second vertebra from a lateral approach, the second component having a second articular surface for cooperating with the first articular surface to permit articulating motion between the first and second components. The subject matter of the present invention differs from this prior art at least in that the ratio of length to width for each of the first and second components is in the range of 1.3:1 to 1.7:1.

FR 2 734 148 A1 discloses an intervertebral prosthesis with first and second components having a ratio of length to width in the range of about 1.2:1 to 1.5:1. The components have, however, an elliptical shape and no straight and parallel longitudinal sides

### Summary

The present invention provides a prosthetic device according to independent claim 1. Further embodiments of the prosthetic device according to the present invention are described in the dependent claims.

### Brief Description of the Drawings

Fig. 1 is a lateral view of a portion of a vertebral column.
Fig. 2 is a lateral view of a pair of adjacent vertebral bodies defining an intervertebral space.
Fig. 3 is a perspective view of an intervertebral prosthetic disc according to one embodiment of the present disclosure.
Fig. 4 is a lateral view of the intervertebral prosthetic disc of Fig. 3.
Fig. 5 is a longitudinal view of the intervertebral prosthetic disc of Fig. 3.
Fig. 6 is a perspective view of a portion of the intervertebral prosthetic disc shown exploded from a vertebral body.
Fig. 7a is a plan view of a portion of the intervertebral prosthetic disc shown laterally disposed in an intervertebral space.
Fig. 7b is a lateral view of the arrangement of Fig. 7a.
Fig. 8a is a plan view of a portion of the intervertebral prosthetic disc shown laterally disposed in an offset manner in an intervertebral space.
Fig. 8b is a lateral view of the arrangement of Fig. 8a.
Fig. 9a is a plan view of a portion of the intervertebral prosthetic disc shown laterally disposed in an offset manner in an intervertebral space.
Fig. 9b is a lateral view of the arrangement of Fig. 9a.
Fig. 10 is a plan view of a portion of an alternative intervertebral disc prosthesis as such not disclosing an embodiment according to the present invention (the claimed length to width ratio not being explicity mentionned), but describing certain aspects of the invention.

### Description

This disclosure relates generally to intervertebral disc prostheses for lateral insertion and, in some instances, laterally offset insertion. For the purposes of promoting an understanding of the principles of the disclosure, reference will now be made to the embodiments, or examples, illustrated in the drawings and specific language will be used to describe the same. It will nevertheless be understood that no limitation of the scope of the disclosure is thereby intended. Any alterations and further modifications in the described embodiments, and any further applications of the principles of the disclosure as described herein are contemplated as would normally occur to one skilled in the art to which this disclosure relates. As such, individual features of separately described embodiments can be combined to form additional embodiments.

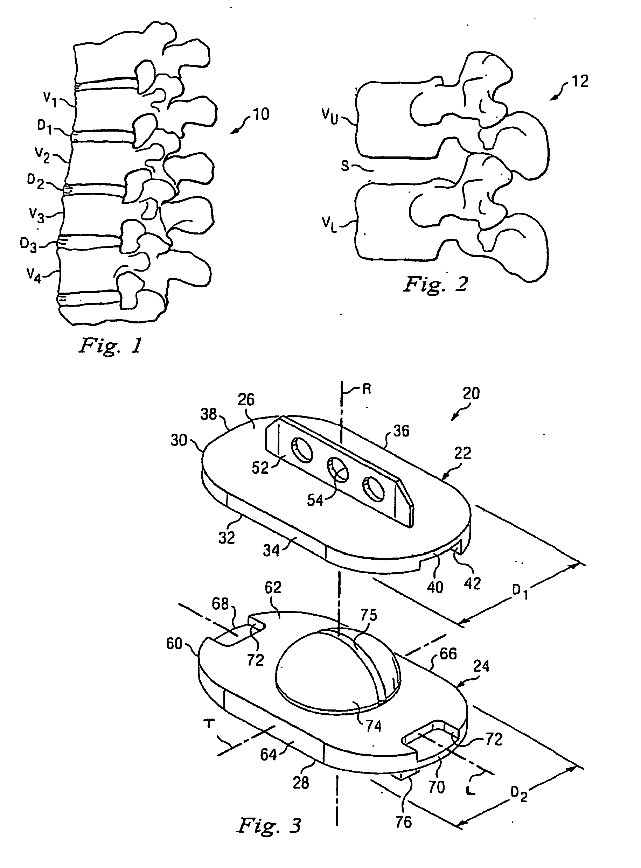

Referring now to Fig. 1, shown therein is a lateral view of a portion of a spinal column 10, illustrating a group of adjacent upper and lower vertebrae V1, V2, V3, V4 separated by natural intervertebral discs D1, D2, D3. The illustration of four vertebrae is only intended as an example. Another example would be a sacrum and one vertebrae.

Referring now to Fig. 2, for the sake of further example, two of the vertebrae will be discussed, designated as a spinal segment 12 including a lower vertebrae V_{L} and an upper vertebrae V_{U}. In one embodiment, some or all of the natural disc that would have been positioned between the two vertebrae V_{L}, V_{U} is typically removed via a discectomy or a similar surgical procedure, the details of which would be known to one of ordinary skill in the art. Removal of the diseased or degenerated disc results in the formation of an intervertebral space S between the upper and lower vertebrae V_{U}, V_{L}.

Referring now to Figs. 3-5, shown therein is one embodiment of an intervertebral prosthetic disc 20 for lateral insertion into the intervertebral space S (Fig. 2). In one embodiment, the prosthetic disc 20 provides for articulating motion, thereby restoring motion to the spinal segment defined by the upper and lower vertebrae V_{U}, V_{L}. The prosthetic disc 20 extends generally along a transverse axis T corresponding to the anterior-posterior aspect of spinal segment 12 and along longitudinal axis L corresponding to the lateral aspect of spinal segment 12.

The prosthetic disc 20 includes a first articular component 22 and a second articular component 24. The articular components 22, 24 cooperate to form the prosthetic disc 20 which is sized and configured for disposition within the intervertebral space S (Fig. 2) between adjacent vertebral bodies V_{U}, V_{L} (Fig. 2). The prosthetic disc 20 provides relative pivotal and rotational movement between the adjacent vertebral bodies to maintain or restore motion substantially similar to the normal bio-mechanical motion provided by a natural intervertebral disc. More specifically, the articular components 22, 24 are permitted to pivot relative to one another about a number of axes, including lateral or side-to-side pivotal movement about axis T and anterior-posterior pivotal movement about axis L. It should be understood that in one embodiment of the disclosure, the articular components 22, 24 are permitted to pivot relative to one another about any axis that lies in a plane that intersects longitudinal axis L and transverse axis T. Furthermore, the articular components 22, 24 are permitted to rotate relative to one another about a rotational axis R. Although the prosthetic disc 20 has been illustrated and described as providing a specific combination of articulating motion, it should be understood that other combinations of articulating movement are also possible, such as, for example, relative translational or linear motion, and such movement is contemplated as falling within the scope of the present disclosure.

Although the articular components 22, 24 of prosthetic disc 20 may be formed from a wide variety of materials, in one embodiment of the disclosure, the articular components 22, 24 are formed of a cobalt-chrome-molybdenum metallic alloy (ASTM F-799 or F-75). However, in alternative embodiments of the disclosure, the articular components 22, 24 may be formed of other materials such as titanium or stainless steel, a polymeric material such as polyethylene, or any other biocompatible material that would be apparent to one of ordinary skill in the art.

The articular components 22, 24 each include a bearing surface 26, 28, respectively, that may be positioned in direct contact with vertebral bone and is preferably coated with a bone-growth promoting substance, such as, for example, a hydroxyapatite coating formed of calcium phosphate. Additionally, the bearing surfaces 26, 28 of the articular components 22, 24, respectively, may be roughened prior to being coated with the bone-growth promoting substance to further enhance bone on-growth. Such surface roughening may be accomplished by way of, for example, acid etching, knurling, application of a bead coating, or other methods of roughening that would occur to one of ordinary skill in the art.

Articular component 22 includes a support plate 30 having an articular surface 32 and the opposite bearing surface 26. Support plate 30 is sized and shaped to provide a technically feasible maximum amount of endplate support for the adjacent vertebra V_{U} (Fig. 2) while minimizing the lateral window associated with insertion of the prosthetic disc 20. For example, the support plate 30 may be shaped such that longitudinal sides 34, 36 of the support plate 30 are substantially parallel and are separated by a distance D1, which corresponds to the lateral window for insertion as will be further described. Additionally, the lateral sides 38, 40 of the support plate 30 may take a curved configuration to correspond to the curvature of the endplate of the adjacent vertebra V_{U} (Fig. 2). As can be appreciated, the longitudinal sides 34, 36 are elongated relative to the lateral sides 38, 40 to facilitate lateral insertion of the prosthetic disc 20 into the disc space S (Fig. 2). In some embodiments, the ratio of the length of the longitudinal sides 34 or 36 to the width D1 is about 1.3:1 to 1.7:1, with a ratio of about 1.5:1 for the disclosed embodiment.

The support plate 30 may include one or more notches 42 or other types of indicia for receiving or engaging with a corresponding portion of a surgical instrument (not shown) to aid in the manipulation and insertion of the prosthetic disc 20 within the intervertebral space S (Fig. 2) between the adjacent vertebral bodies V_{U}, V_{L} (Fig. 2).

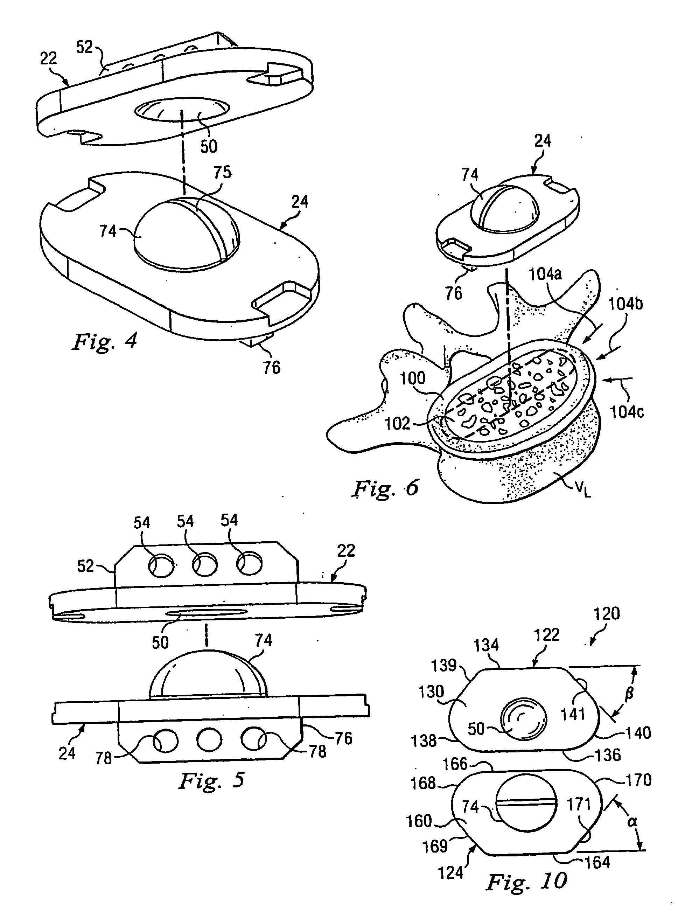

Referring to Fig. 4, in one embodiment of the disclosure, the articular component 22 includes a recess 50. In one embodiment, the recess 50 has a concave shape, and is configured as a spherical-shaped socket. However, it should be understood that other configurations of the recess 50 are also contemplated, such as, for example, cylindrical, elliptical or other arcuate configurations or possibly non-arcuate configurations. The remaining portion of the articular surface 32 can be angled or otherwise configured to facilitate the insertion and/or use of the prosthesis.

Although the concave recess 50 is illustrated as having a generally smooth, uninterrupted articular surface, it should be understood that a surface depression or cavity may be defined along a portion of the recess 50 to provide a means for clearing out matter, such as particulate debris, that is disposed between the abutting articular components 22, 24.

A flange member or keel 52 extends from the bearing surface 26 and is configured for disposition within a preformed opening in the adjacent vertebral endplate. As with the bearing surface 26, the keel 52 may be coated with a bone-growth promoting substance, such as, for example, a hydroxyapatite coating formed of calcium phosphate. Additionally, the keel 52 may be roughened prior to being coated with the bone-growth promoting substance to further enhance bone on-growth. In one embodiment, the keel 52 extends along the axis L and is substantially centered along the bearing surface 26. However, it should be understood that other positions and orientations of the keel 52 are also contemplated.

In one embodiment, the keel 52 longitudinally extends along a substantial portion of the articular component 22. Such an embodiment would accommodate insertion of the prosthetic joint 20 using a lateral approach as opposed to, for example, an anterior approach. In a further embodiment, the keel 52 may be angled, tapered, or configured in some other shape to facilitate the functional demands of the keel. In still another embodiment, the keel 52 may be configured as a winged keel, including a lateral portion (not shown) extending across the main body portion of keel 52.

In one embodiment, the keel 52 includes three openings 54 extending therethrough to facilitate bone through-growth to enhance fixation to the adjacent vertebral bodies V_{U}, V_{L} (Fig. 2). However, it should be understood that any number of openings 54 may be defined through the keel 52, including a single opening or two or more openings. It should also be understood that the openings 54 need not necessarily extend entirely through the keel 52, but may alternatively extend partially therethrough. It should further be understood that the keel 52 need not necessarily define any openings 54 extending either partially or entirely therethrough. Additionally, although the openings 54 are illustrated as having a circular configuration, it should be understood that other sizes and configurations of openings 54 are also contemplated.

Articular component 24 includes a support plate 60 having an articular surface 62 and the opposite bearing surface 28. Support plate 60 is sized and shaped to provide a technically feasible maximum amount of endplate support for the adjacent vertebra V_{L} (Fig. 2) while minimizing the lateral window associated with insertion of the prosthetic disc 20. For example, the support plate 60 may be shaped such that longitudinal sides 64, 66 of the support plate 30 are substantially parallel and are separated by the distance D2, which corresponds to the lateral window for insertion as will be further described. Additionally, the lateral sides 68, 70 of the support plate 60 may take a curved configuration to correspond to the curvature of the endplate of the adjacent vertebra V_{L} (Fig. 2). As can be appreciated, the longitudinal sides 64, 66 are elongated relative to the lateral sides 68, 70 to facilitate lateral insertion of the prosthetic disc 20 into the disc space S (Fig. 2). In some embodiments, the ratio of the length of the longitudinal sides 64 or 66 to the width D2 is about 1.3:1 to 1.7:1, with a ratio of about 1.5:1 for the disclosed embodiment.

In some embodiments, support plates 60 and 30 are symmetrical in shape, with distance D1 equal to distance D2. In other embodiments, the plates 60, 30 may be of different sizes and shapes to accommodate different requirements. For example, in some embodiments, distance D1 does not equal distance D2.
The support plate 60 may include one or more notches 72 or other types of indicia for receiving or engaging with a corresponding portion of a surgical instrument (not shown) to aid in the manipulation and insertion of the prosthetic joint 20 within the intervertebral space S (Fig. 2) between the adjacent vertebral bodies V_{U}, V_{L} (Fig. 2). In one embodiment, the notches 72 are shaped in a manner similar to that of the notches 42.

The notches 42, 72 may be formed to selectively lock or otherwise engage with an insertion-type surgical instrument (not shown). The surgical instrument is preferably configured to hold the articular components 24, 24 at a predetermined orientation and spatial relationship relative to one another during manipulation and insertion of the prosthetic disc 20, and to release the articular components 24, 24 once properly positioned between the adjacent vertebrae. In other embodiments, a combination of holes, apertures, and other mechanisms can be used to engage with various surgical instruments.

In one embodiment of the disclosure, the articular component 22 includes a projection 74 having a convex shape, which may be configured as a spherical-shaped ball (half of which is shown). It should be understood that other configurations of the projection 74 are also contemplated, such as, for example, cylindrical, elliptical or other arcuate configurations or possibly non-arcuate configurations. It should also be understood that the remaining portion of articular component 22 may take on planar or non-planar configurations, such as, for example, an angular or conical configuration extending about the projection 74.

A surface depression or cavity 75 may be defined along a portion of the projection 74 to provide a means for clearing out matter, such as particulate debris, that is disposed between the abutting articular components 22, 24. Of course, in other embodiments, the convex articular surface of the projection 74 may alternatively define a generally smooth, uninterrupted articular surface. In another embodiment, each of the convex projection 74 and the concave recess 50 may define a surface depression to facilitate removal of particulate matter disposed between the abutting articular components 22, 24.

A flange member or keel 76 extends from the bearing surface 28 and is configured for disposition within a preformed opening in the adjacent vertebral endplate. As with the bearing surface 28, the keel 76 may be coated with a bone-growth promoting substance, such as, for example, a hydroxyapatite coating formed of calcium phosphate. Additionally, the keel 76 may be roughened prior to being coated with the bone-growth promoting substance to further enhance bone on-growth. In one embodiment, the keel 76 extends along the axis L and is substantially centered along the bearing surface 62. However, it should be understood that other positions and orientations of the keel 76 are also contemplated.

In one embodiment, the keel 76 longitudinally extends along a substantial portion of the articular component 24. Such an embodiment would accommodate insertion of the prosthetic disc 20 using a lateral approach as opposed to, for example, an anterior approach. In a further embodiment, the keel 76 may be angled, tapered, or configured in some other shape to facilitate the functional demands of the keel. In still another embodiment, the keel 76 may be configured as a winged keel, including a lateral portion (not shown) extending across the main body portion of keel 76.

In one embodiment, the keel 76 includes three openings 78 extending therethrough to facilitate bone through-growth to enhance fixation to the adjacent vertebral bodies V_{U}, V_{L} (Fig. 2). However, it should be understood that any number of openings 78 may be defined through the keel 76, including a single opening or two or more openings. It should also be understood that the openings 78 need not necessarily extend entirely through the keel 76, but may alternatively extend partially therethrough. It should further be understood that the keel 76 need not necessarily define any openings 78 extending either partially or entirely therethrough. Additionally, although the openings 78 are illustrated as having a circular configuration, it should be understood that other sizes and configurations of openings 78 are also contemplated.

Referring now to Fig. 6, articular component 24 is shown exploded from the lower vertebral body V_{L}. In this example, the natural endplate associated with the lower vertebral body V_{L} has been removed. However, it is understood that the prosthetic device 20 may be used in situations where the endplate remains intact with the adjacent vertebral body. The vertebral body V_{L} includes an outer ring of cortical bone 100, often referred to as the apophyseal ring. The inner portion of the vertebral body V_{L} comprises cancellous bone 102, which is softer and weaker than the cortical bone of the apophyseal ring. The design of the articular component 24, and therefore the prosthetic device 20, facilitates contact between the prosthetic device and the cortical bone 100 of the apophyseal ring, thereby providing an appropriate bearing surface which prevents subsidence of the prosthetic device into the cancellous bone portion of the lower vertebral body V_{L}.

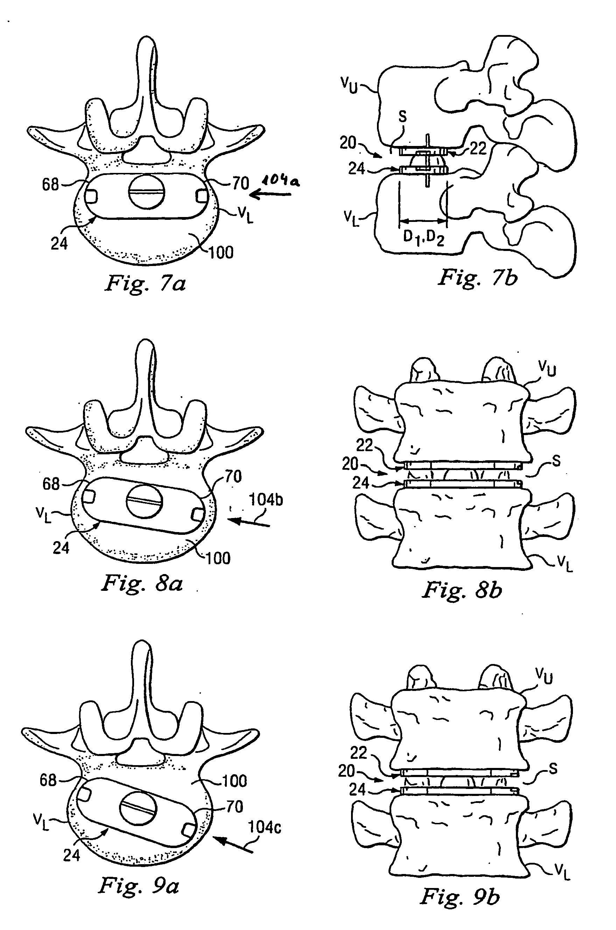

Referring also to Figs. 7-9, the prosthetic disc 20, as represented by the articular component 24, can be inserted from several different lateral approaches 104a, 104b, and 104c. The approach 104a (corresponding with Figs. 7a, 7b) is a direct lateral insertion trajectory, parallel with the axis L (Fig. 3). The approach 104b (corresponding with Figs. 8a, 8b) is an oblique insertion trajectory, 10-degree offset from the axis L. The approach 104c (corresponding with Figs. 9a, 9b) is an oblique insertion trajectory, 20-degree offset from the axis L. The curved shape of the lateral sides 38, 40 and 68, 70 can be chosen to support the different approaches 104a, 104b, 104c so that regardless of the approach, a portion of the corresponding articular components 22, 24 will be suitably positioned above the cortical bone 100 of the apophyseal ring.

For example, referring to Fig. 7a, the articular component 24 is shaped to span the length of the adjacent vertebral body V_{L} such that the lateral edges 68, 70 of the articular component 24 are nearly or substantially flush with the edges of the vertebral body V_{L}. Accordingly, the articular component 24 has sufficient length so that it bears against a technically feasible maximum amount of the cortical bone 100 for a given lateral window. As such, subsidence of the articular component 24 into the vertebral body V_{L} can be prevented.

Turning now to Fig. 7b, the lateral window associated with insertion of the prosthetic device 20 into the intervertebral space S (Fig. 2) is generally shown. As can be appreciated, the size of the lateral window generally corresponds to the amount of trauma imparted to the vertebral region during lateral insertion of prosthetic devices. In some embodiments, the lateral window has a width that is substantially equal to the distances D1, D2 for each of the articular components 22, 24 (Fig. 3). Accordingly, by minimizing the width of the prosthetic device 20, the lateral window associated with its insertion is in turn reduced.

Referring now to Figs. 8a and 8b, the prosthetic device 20 can be inserted from an oblique approach 104b rather than a direct lateral approach 104a as shown in Figs. 6, 7a and 7b. In such embodiments, the width of the prosthetic device 20 defined by the distances D1, D2 between the longitudinal sides 34, 36 defines the lateral window for insertion, which again is minimal. Also, even from an oblique approach, the prosthetic device 20 essentially spans the length of the vertebral bodies V_{U}, V_{L} to bear against an optimal amount of cortical bone of the apophyseal ring.

Figs. 9a and 9b are depicted by way of further example to illustrate that the prosthetic device 20 of the present disclosure can be inserted from the more pronounced oblique angle 104c (Fig. 6) relative to the example of Figs. 8a and 8b.

Referring to Fig. 10, a portion of an alternative prosthetic device is generally referred to by reference numeral 120. The prosthetic disc 120 includes a first articular component 122 and a second articular component 124. The articular components 122, 124 cooperate to form the prosthetic disc 120 which is sized and configured for disposition within the intervertebral space S (Fig. 2) between adjacent vertebral bodies V_{U}, V_{L} (Fig. 2). The prosthetic disc 120 provides relative pivotal and rotational movement between the adjacent vertebral bodies to maintain or restore motion substantially similar to the normal bio-mechanical motion provided by a natural intervertebral disc. More specifically, the articular components 122, 124 are permitted to pivot relative to one another about a number of axes, including lateral or side-to-side pivotal movement about a transverse axis and anterior-posterior pivotal movement about longitudinal axis. It should be understood that in one embodiment of the disclosure, the articular components 122, 124 are permitted to pivot relative to one another about any axis that lies in a plane that intersects the longitudinal and transverse axes. Furthermore, the articular components 122, 124 are permitted to rotate relative to one another about a rotational axis. Although the prosthetic disc 120 has been illustrated and described as providing a specific combination of articulating motion, it should be understood that other combinations of articulating movement are also possible, such as, for example, relative translational or linear motion, and such movement is contemplated as falling within the scope of the present disclosure.

Articular component 122 includes a support plate 130 shaped such that longitudinal sides 134, 136 of the support plate 130 are substantially parallel and are separated by the distance D1. Additionally, the lateral sides of the support plate 130 include curved portions 138, 140 that correspond to the curvature of the endplate of the adjacent vertebra V_{U} (Fig. 2) and straight portions 139, 141. The straight portions 139, 141 are set an angle β of about 60 degrees from the side 134. As can be appreciated, the longitudinal sides 134, 136 are elongated relative to the lateral sides to facilitate lateral insertion of the prosthetic disc 120 into the disc space S (Fig. 2).

Articular component 124 includes a support plate 160 such that longitudinal sides 164, 166 of the support plate 130 are substantially parallel and are separated by the distance D2. Additionally, the lateral sides of the support plate 160 include curved portions 168, 170 that correspond to the curvature of the endplate of the adjacent vertebra V_{U} (Fig. 2) and straight portions 169, 171. The straight portions 169, 171 are set an angle a of about 60 degrees from the side 164. As can be appreciated, the longitudinal sides 164, 166 are elongated relative to the lateral sides to facilitate lateral insertion of the prosthetic disc 120 into the disc space S (Fig. 2).

Accordingly, it is understood that several modifications, changes and substitutions are intended in the foregoing disclosure and, in some instances, some features of the disclosure will be employed without a corresponding use of other features. For example, features such as the keels 52, 76 may not be used in some embodiments. Also, instead of two components with a single articulating surface as described, alternative embodiments may utilize dual articulating surfaces, such as disclosed in U.S. Publication Number 2002/0035400. It is also understood that all spatial references, such as "inner," "outer," "proximal," and "distal" are for illustrative purposes only and can be varied within the scope of the disclosure. Accordingly, it is appropriate that the appended claims be construed broadly and in a manner consistent with the scope of the disclosure.

## Claims

1. A prosthetic device (20, 120) for lateral insertion into an intervertebral space (S) between a first vertebral body (V_{L}) and a second vertebral body (V_{U}) forming a spinal segment (12), the prosthetic device (20, 120) - when being inserted into the intervertebral space (S) - extending along a transverse axis (T) corresponding to an anterior-posterior direction of the spinal segment (12) and along a longitudinal axis (L) corresponding to a lateral direction of the spinal segment (12), the prosthetic device (20, 120) comprising a first component (22, 122) for engaging the first vertebral body (V_{U}), a second component (24, 124) for engaging the second vertebral body (V_{L}), and an articulation member (50, 74) positioned between the first and second components (22, 24, 122, 124) to permit articulating motion between the first and second components (22, 24, 122, 124), wherein the first and second components (22, 24, 122, 124)
respectively comprise a support plate (30, 60, 130, 160) with relatively straight and parallel longitudinal sides (34, 36, 64, 66, 134, 136, 164, 166) extending parallel to the longitudinal axis (L) of the prosthetic device (20, 120),
each have a length that - when the prosthetic device (20, 120) is inserted into the intervertebral space (S) - extends upon cortical bone of opposing first and second sides of an apophyseal ring (100) of the corresponding vertebral body (V_{L}, V_{U}), the first and second sides being opposed in the lateral direction of the spinal segment (12), and
each have a width that is smaller than the length and such that it does not extend upon opposing third and fourth sides of the apophyseal ring (100) of the corresponding vertebral body (V_{L}, V_{U}), the third and fourth sides being opposed in the anterior-posterior direction of the spinal segment (12), wherein a ratio of length to width for each of the first and second components (22, 24, 122, 124) is in the range of 1.3:1 to 1.7:1.

2. The prosthetic device (20) of claim 1 wherein the respective support plate (30, 60) has curved lateral sides (38, 40, 68, 70).

3. The prosthetic device (120) of claim 1 wherein the respective support plate (130, 160) has at least one lateral side having a curved portion (138, 140) and a straight portion (139, 141).

4. The prosthetic device (20, 120) of claim 1 wherein the articulation member (50, 74) includes a convex portion (74) connected to the second component (24, 124) and a concave portion (50) connected to the first component (22, 122).

5. The prosthetic device (20, 120) of claim 1 wherein a spherical shaped protrusion (74) extends from the support plate (60, 160) of the second component (24, 124), and wherein a recess is formed in the support plate (30, 130) of the first component (22, 122), such that the spherical shaped protrusion (74) and the recess (50) comprise the articulation member.

6. The prosthetic device (20) of claim 1 wherein the first component (22) includes a first longitudinally-extending flange (52) for engaging the first vertebral body (V_{U}) from a lateral approach, and wherein the second component (24) includes a second longitudinally-extending flange (76) for engaging the second vertebral body (V_{L}) from the lateral approach.

7. The prosthetic device (20) of claim 6 wherein the first flange (52) is offset relative to the second flange (76) to accommodate insertion into the first and second vertebral bodies (V_{L}, V_{U}) being in a spondylosed relationship.

8. The prosthetic device (20) of claim 6 wherein the first flange (52) is aligned with the second flange (76) to accommodate insertion into the first and second vertebral bodies (V_{L}, V_{U}) being in an aligned relationship.

9. The prosthetic device (20) of claim 1 wherein the first component (22) further comprises a first bearing surface (26) adapted to engage the apophyseal ring (100) of the first vertebral body (V_{U}).

10. The prosthetic device (20) of claim 9 wherein the first component (22) includes a first longitudinally-extending flange (52) for engaging the first vertebral body (V_{U}) from a lateral approach and wherein the first flange (52) extends along a substantial portion of the first bearing surface (26).

11. The prosthetic device (20) of claim 1 wherein the second component (24) further comprises a second bearing surface (28) adapted to engage the apophyseal ring (100) of the second vertebral body (V_{L}).

12. The prosthetic device (20) of claim 11 wherein the second component (24) includes a second longitudinally-extending flange (76) for engaging the second vertebral body (V_{L}) from a lateral approach and wherein the second flange (76) extends along a substantial portion of the second bearing surface (28).

13. The prosthetic device (20) of claim 6 wherein the first and second flanges (52, 76) each comprise at least one hole (54, 78) therethrough.

14. The prosthetic device (20, 120) of claim 6 wherein the first and second flanges (52, 76) are each coated with a bone-growth promoting substance.

15. The prosthetic device (20) of claim 1 wherein the first component (22) further comprises a first bearing surface (26) adapted to engage the apophyseal ring (100) of the first vertebral body (V_{U}), and wherein the second component (24) further comprises a second bearing surface (28) adapted to engage the apophyseal ring (100) of the second vertebral body (V_{L}), and wherein the first and second bearing surfaces (26, 28) are each coated with a bone-growth promoting substance.

16. The prosthetic device (20, 120) of claim 1 wherein the first and second components (22, 24, 122, 124) and articulation member (50, 74) are formed of a cobalt-chrome-molybdenum metallic alloy.

17. The prosthetic device (20) of claim 1 wherein the first and second components (22, 24) each comprise at least one notch (42, 72) formed laterally therein for receiving a surgical instrument.

18. The prosthetic device (20, 120) of claim 1 wherein the ratio of length to width for each of the first and second components (22, 24, 122, 124) is in the range of about 1.5:1.

## Patentansprüche

1. Eine prothetische Vorrichtung (20, 120) zum lateralen Einsetzen in einen Zwischenwirbelraum (S) zwischen einem ersten Wirbelkörper (V_{L}) und einem zweiten Wirbelkörper (V_{U}), welche ein spinales Segment (12) formen, wobei die prothetische Vorrichtung (20, 120) - wenn sie in den Zwischenwirbelraum (S) eingesetzt ist - sich entlang einer Querachse (T) entsprechend einer anterior-posterior-Richtung von dem spinalen Segment (12) sowie entlang einer Längsachse (L) entsprechend einer lateralen Richtung von dem spinalen Segment (12) erstreckt, und wobei die prothetische Vorrichtung (20, 120) eine erste Komponente (22, 122) zum Eingriff mit dem ersten Wirbelkörper (V_{U}), eine zweite Komponente (24, 124) zum Eingriff mit dem zweiten Wirbelkörper (V_{L}) und ein Gelenkteil (50, 74) aufweist, welches zwischen der ersten und der zweiten Komponente (22, 24, 122, 124) positioniert ist, um zwischen der ersten und der zweiten Komponente (22, 24, 122, 124) eine Gelenkbewegung zuzulassen, wobei die erste und die zweite Komponente (22, 24, 122, 124)
jeweils eine Stützplatte (30, 60, 130, 160) mit relativ geraden und parallelen Längsseiten (34, 36, 64, 66, 134, 136, 164, 166) aufweisen, welche sich parallel zu der Längsachse (L) der prothetischen Vorrichtung (20, 120) erstrecken,
jeweils eine Länge haben, welche - wenn die prothetische Vorrichtung (20, 120) in den Zwischenwirbelraum (S) eingesetzt ist - sich auf kortikalen Knochen von gegenüberliegender erster und zweiter Seite eines Apophysenrings (100) des entsprechenden Wirbelkörpers (V_{L}, V_{U}) erstreckt, wobei die erste Seite und die zweite Seite sich in lateraler Richtung von dem spinalen Segment (12) gegenüberliegen, und
jeweils eine Breite haben, welche kleiner ist als die *Länge und derart, dass sie sich nicht auf eine dritte und vierte Seite von dem Aphophysenring (100) von dem entsprechenden Wirbelkörper (V_{L}, V_{U}), welche sich gegenüberliegen, erstreckt, wobei die dritte Seite und die vierte Seite sich in der anterior-posterior-Richtung von dem spinalen Segment (12) gegenüberliegen,
wobei ein Verhältnis von Länge zu Breite für jede von der ersten und der zweiten Komponente (22, 24, 122, 124) in dem Bereich von 1,3:1 bis 1,7:1 ist.

2. Die prothetische Vorrichtung (20) nach Anspruch 1, wobei die jeweilige Stützplatte (30, 60) gekrümmte laterale Seiten (38, 40, 68, 70) aufweist.

3. Die prothetische Vorrichtung (120) nach Anspruch 1, wobei die jeweilige Stützplatte (130, 160) zumindest eine laterale Seite mit einem gekrümmten Abschnitt (138, 140) und einem geraden Abschnitt (139, 141) hat.

4. Die prothetische Vorrichtung (20, 120) nach Anspruch 1, wobei das Gelenkteil (50, 74) einen konvexen Abschnitt (74) aufweist, welcher mit der zweiten Komponente (24, 124) verbunden ist, sowie einen konkaven Abschnitt (50), welcher mit der ersten Komponente (22, 122) verbunden ist.

5. Die prothetische Vorrichtung (20, 120) nach Anspruch 1, wobei sich ein sphärisch geformter Vorsprung (74) von der Stützplatte (60, 160) der zweiten Komponente (24, 124) erstreckt, und wobei eine Aussparung in der Stützplatte (30, 130) der ersten Komponente (22, 122) geformt ist, sodass der sphärisch geformte Vorsprung (74) und die Aussparung (50) das Gelenkteil ausbilden.

6. Die prothetische Vorrichtung (20) nach Anspruch 1, wobei die erste Komponente (22) einen ersten, sich in Längsrichtung erstreckenden Flansch (52) zum Engreifen mit dem ersten Wirbelkörper (V_{U}) von einer lateralen Herangehensweise aus aufweist, und wobei die zweite Komponente (24) einen zweiten, sich in Längsrichtung erstreckenden Flansch (76) zum Eingreifen mit dem zweiten Wirbelkörper (V_{L}) von der lateralen Herangehensweise aus aufweist.

7. Die prothetische Vorrichtung (20) nach Anspruch 6, wobei der erste Flansch (52) relativ zu dem zweiten Flansch (76) versetzt ist, um einem Einsetzen in den ersten und den zweiten Wirbelkörper (V_{L}, V_{U}), welche in einer Spondylose-Beziehung sind, entgegenzukommen.

8. Die prothetische Vorrichtung (20) nach Anspruch 6, wobei der erste Flansch (52) mit dem zweiten Flansch (76) ausgerichtet ist bzw. fluchtet, um ein Einsetzen in den ersten und den zweiten Wirbelkörper (V_{L}, V_{U}), welche in einer ausgerichteten Beziehung sind, entgegenzukommen.

9. Die prothetische Vorrichtung (20) nach Anspruch 1, wobei die erste Komponente (22) ferner eine erste Tragfläche (26) hat, welche angepasst ist, um mit dem Apophysenring (100) von dem ersten Wirbelkörper (V_{U}) einzugreifen.

10. Die prothetische Vorrichtung (20) nach Anspruch 9, wobei die erste Komponente (22) einen ersten, sich in Längsrichtung erstreckenden Flansch (52) zum Eingreifen mit dem ersten Wirbelkörper (V_{U}) von einer lateralen Herangehensweise aus hat, und wobei der erste Flansch (52) sich entlang eines wesentlichen Abschnitts von der ersten Tragfläche (26) erstreckt.

11. Die prothetische Vorrichtung (20) nach Anspruch 1, wobei die zweite Komponente (24) ferner eine zweite Tragfläche (28) aufweist, welche angepasst ist, um mit dem Apophysenring (100) von dem zweiten Wirbelkörper (V_{L}) einzugreifen.

12. Die prothetische Vorrichtung (20) nach Anspruch 11, wobei die zweite Komponente (24) einen zweiten, sich in Längsrichtung erstreckenden Flansch (76) zum Eingriff mit dem zweiten Wirbelkörper (V_{L}) von einer lateralen Herangehensweise aus hat, und wobei der zweite Flansch (76) sich entlang eines wesentlichen Abschnitts von der zweiten Tragfläche (28) erstreckt.

13. Die prothetische Vorrichtung (20) nach Anspruch 6, wobei der erste und der zweite Flansch (52, 76) jeweils zumindest ein Loch (54, 78) dort hindurch aufweisen.

14. Die prothetische Vorrichtung (20, 120) nach Anspruch 6, wobei der erste und der zweite Flansch (52, 76) jeweils beschichtet sind mit einer Knochen-Wachstum-fördernden Substanz.

15. Die prothetische Vorrichtung (20) nach Anspruch 1, wobei die erste Komponente (22) ferner aufweist eine erste Tragfläche (26), welche angepasst ist, um mit dem Apophysenring (100) von dem ersten Wirbelkörper (V_{U}) einzugreifen, und wobei die zweite Komponente (24) ferner aufweist eine zweite Tragfläche (28), welche angepasst ist, um mit dem Apophysenring (100) von dem zweiten Wirbelkörper (V_{L}) einzugreifen, und wobei die erste und die zweite Tragfläche (26, 28) jeweils mit einer Knochen-Wachstum-fördernden Substanz beschichtet sind.

16. Die prothetische Vorrichtung (20, 120) nach Anspruch 1, wobei die erste und die zweite Komponente (22, 24, 122, 124) sowie das Gelenkteil (50, 74) aus einer Kobalt-Chrom-Molybdän-Metalllegierung geformt sind.

17. Die prothetische Vorrichtung (20) nach Anspruch 1, wobei die erste und die zweite Komponente (22, 24) jeweils zumindest eine Aussparung (42, 72) haben, welche lateral darin geformt ist zum Aufnehmen eines chirurgischen Instruments.

18. Die prothetische Vorrichtung (20, 120) nach Anspruch 1, wobei das Verhältnis von Länge zu Breite für jede von der ersten und der zweiten Komponente (22, 24, 122, 124) in dem Bereich von ungefähr 1,5:1 ist.

## Revendications

1. Dispositif prothétique (20, 120) pour une insertion latérale dans un espace intervertébral (S) entre un premier corps vertébral (V_{L}) et un second corps vertébral (v_{U}) formant un segment médullaire (12), le dispositif prothétique (20, 120) - lorsqu'inséré dans l'espace intervertébral (S) - s'étendant le long d'un axe transversal (T) correspondant à une direction antéro-postérieure du segment médullaire (12) et le long d'un axe longitudinal (L) correspondant à une direction latérale du segment médullaire (12), le dispositif prothétique (20, 120) comprenant un premier composant (22, 122) destiné à se mettre en prise avec le premier corps vertébral (v_{U}), un second composant (24, 124) destiné à se mettre en prise avec le second corps vertébral (v_{L}), et un élément d'articulation (50, 74) positionné entre les premier et second composants (22, 24, 122, 124) pour permettre un mouvement articulatoire entre les premier et second composants (22, 24, 122, 124), dans laquelle les premier et second composants (22, 24, 122, 124)
comprennent respectivement une plaque de support (30, 60, 130, 160) avec des côtés longitudinaux relativement droits et parallèles (34, 36, 64, 66, 134, 136, 164, 166) s'étendant parallèlement à l'axe longitudinal (L) du dispositif prothétique (20, 120),
ont chacun une longueur qui - lorsque le dispositif prothétique (20, 120) est inséré dans l'espace intervertébral (S) - s'étend sur l'os cortical des premier et deuxième côtés opposés d'une anneau apophysaire (100) du corps vertébral (v_{L}, v_{U}) correspondant, les premier et second côtés étant opposés dans la direction latérale du segment médullaire (12), et
ont chacun une largeur qui est inférieure à la longueur et telle qu'elle ne s'étend pas sur les troisième et quatrième côtés opposés de l'anneau apophysaire (100) du corps vertébral (v_{L}, v_{U}) correspondant, les troisième et quatrième côtés étant opposés dans la direction antéro-postérieure du segment médullaire (12),
dans laquelle un rapport longueur à largeur pour chacun des premier et deuxième composants (22, 24, 122, 124) est dans la plage de 1,3:1 à 1,7:1.

2. Dispositif prothétique (20) selon la revendication 1, dans lequel la plaque de support (30, 60) respective présente des côtés latéraux incurvés (38, 40, 68, 70).

3. Dispositif prothétique (120) selon la revendication 1, dans lequel la plaque de support (130, 160) respective présente au moins un côté latéral ayant une partie incurvée (138, 140) et une partie droite (139, 141).

4. Dispositif prothétique (20, 120) selon la revendication 1, dans lequel l'élément d'articulation (50, 74) comprend une partie convexe (74) reliée au second composant (24, 124) et une partie concave (50) reliée au premier composant (22, 122).

5. Dispositif prothétique (20, 120) selon la revendication 1, dans lequel une saillie de forme sphérique (74) s'étend à partir de la plaque de support (60, 160) du second composant (24, 124), et dans lequel un évidement est formé dans la plaque de support (30, 130) du premier composant (22, 122), de telle sorte que la saillie de forme sphérique (74) et l'évidement (50) comprennent l'élément d'articulation.

6. Dispositif prothétique (20) selon la revendication 1, dans lequel le premier composant (22) comprend un premier rebord s'étendant longitudinalement (52) destiné à se mettre en prise avec le premier corps vertébral (v_{U}) depuis une approche latérale, et dans lequel le second composant (24) comprend un second rebord s'étendant longitudinalement (76) destiné à se mettre en prise avec le second corps vertébral (v_{L}) depuis l'approche latérale.

7. Dispositif prothétique (20) selon la revendication 6, dans lequel le premier rebord (52) est décalé par rapport au second rebord (76) pour permettre l'insertion dans les premier et second corps vertébraux (v_{L}, v_{U}) se trouvant dans une relation spondylosée.

8. Dispositif prothétique (20) selon la revendication 6, dans lequel le premier rebord (52) est aligné avec le second rebord (76) pour permettre l'insertion dans les premier et second corps vertébraux (v_{L}, v_{U}) se trouvant dans une relation alignée.

9. Dispositif prothétique (20) selon la revendication 1, dans lequel le premier composant (22) comprend en outre une première surface d'appui (26) conçue pour se mettre en prise avec l'anneau apophysaire (100) du premier corps vertébral (v_{U}).

10. Dispositif prothétique (20) selon la revendication 9, dans lequel le premier composant (22) comprend un premier rebord s'étendant longitudinalement (52) destiné à se mettre en prise avec le premier corps vertébral (v_{U}) depuis une approche latérale, et dans lequel le premier rebord (52) s'étend le long d'une partie substantielle de la première surface d'appui (26).

11. Dispositif prothétique (20) selon la revendication 1, dans lequel le second composant (24) comprend en outre une seconde surface d'appui (28) conçue pour se mettre en prise avec l'anneau apophysaire (100) du second corps vertébral (v_{L}).

12. Dispositif prothétique (20) selon la revendication 11, dans lequel le second composant (24) comprend un second rebord s'étendant longitudinalement (76) destiné à se mettre en prise avec le second corps vertébral (v_{L}) depuis une approche latérale, et dans lequel le second rebord (76) s'étend le long d'une partie substantielle de la seconde surface d'appui (28).

13. Dispositif prothétique (20) selon la revendication 6, dans lequel les premier et second rebords (52, 76) comprennent chacun au moins un trou (54, 78) les traversant.

14. Dispositif prothétique (20, 120) selon la revendication 6, dans lequel les premier et second rebords (52, 76) sont chacun revêtus d'une substance favorisant la croissance osseuse.

15. Dispositif prothétique (20) selon la revendication 1, dans lequel le premier composant (22) comprend en outre une première surface d'appui (26) conçue pour se mettre en prise avec l'anneau apophysaire (100) du premier corps vertébral (v_{U}), et dans lequel le second composant (24) comprend en outre une seconde surface d'appui (28) conçue pour se mettre en prise avec l'anneau apophysaire (100) du second corps vertébral (v_{L}), et dans lequel les première et seconde surfaces d'appui (26, 28) sont chacune revêtues d'une substance favorisant la croissance osseuse.

16. Dispositif prothétique (20, 120) selon la revendication 1, dans lequel les premier et second composants (22, 24, 122, 124) et l'élément d'articulation (50, 74) sont formés avec un alliage métallique de cobalt-chrome-molybdène.

17. Dispositif prothétique (20) selon la revendication 1, dans lequel les premier et second composants (22, 24) comprennent chacun au moins une encoche (42, 72) formée latéralement à l'intérieur de ceux-ci pour recevoir un instrument chirurgical.

18. Dispositif prothétique (20, 120) selon la revendication 1, dans lequel le rapport longueur à largeur pour chacun des premier et second composants (22, 24, 122, 124) est dans la plage d'environ 1,5:1.
